(19) Europäisches Patentamt
European Patent Office
Office européen des brevets

(11) Veröffentlichungsnummer : **0 398 101 B1**

# (12) EUROPÄISCHE PATENTSCHRIFT

(45) Veröffentlichungstag der Patentschrift : **04.08.93 Patentblatt 93/31**

(51) Int. Cl.$^5$ : **C07C 237/06,** C07C 229/16, C10L 1/22

(21) Anmeldenummer : **90108499.6**

(22) Anmeldetag : **07.05.90**

## (54) Neue Umsetzungsprodukte von Aminoalkylenpolycarbonsäuren mit sekundären Aminen und Erdölmitteldestillatzusammensetzungen, die diese enthalten.

(30) Priorität : **19.05.89 DE 3916366**

(43) Veröffentlichungstag der Anmeldung : **22.11.90 Patentblatt 90/47**

(45) Bekanntmachung des Hinweises auf die Patenterteilung : **04.08.93 Patentblatt 93/31**

(84) Benannte Vertragsstaaten : **AT BE DE FR GB IT NL SE**

(56) Entgegenhaltungen :
**EP-A- 0 055 355**
**EP-A- 0 188 786**
**DE-A- 2 624 630**
**DE-C- 1 914 756**
**US-A- 4 810 262**
**RÖMPP, CHEMIE-LEXIKON, 8. Auflage, 1979, Seiten 396, 948**

(73) Patentinhaber : **BASF Aktiengesellschaft**
**Carl-Bosch-Strasse 38**
**W-6700 Ludwigshafen (DE)**

(72) Erfinder : **Oppenlaender, Knut, Dr.**
**Otto-Dill-Strasse 23**
**W-6700 Ludwigshafen (DE)**
Erfinder : **Wegner, Brigitte, Dr.**
**Paul-Egell-Strasse 7**
**W-6720 Speyer (DE)**
Erfinder : **Barthold, Klaus, Dr.**
**Paulusbergstrasse 4**
**W-6800 Mannheim 51 (DE)**
Erfinder : **Schwartz, Erich, Dr.**
**Mohnstrasse 37**
**W-6700 Ludwigshafen (DE)**
Erfinder : **Buettner, Egon**
**Wolframstrasse 16**
**W-6700 Ludwigshafen (DE)**
Erfinder : **Schramm, Manfred**
**Buergermeister-Lamberth Strasse 31**
**W-6806 Viernheim (DE)**

EP 0 398 101 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Umsetzungsprodukte von Aminoalkylencarbonsäuren mit sekundären langkettigen Aminen, sowie Mitteldestillate mit einem Gehalt dieser Umsetzungsprodukte, mit verbesserter Kältefließfähigkeit und besserer Dispergierung der ausgeschiedenen Paraffinkristalle.

Mitteldestillate wie z.B. Gasöle, Dieselöle oder Heizöle, die durch Destillation aus Erdölen gewonnen werden, haben je nach Herkunft des Rohöls unterschiedliche Gehalte an Paraffinen. Bei tieferen Temperaturen kommt es zur Ausscheidung fester Paraffine (Trübungspunkt oder Cloud Point, CP). Bei weiterer Abkühlung bilden die plättchenförmigen n-Paraffinkristalle eine "Kartenhausstruktur" und das Mitteldestillat stockt, obwohl der überwiegende Teil des Mitteldestillates noch flüssig ist. Durch die ausgefallenen n-Paraffine im Temperaturgebiet zwischen Trübungspunkt und Stockpunkt (Pour Point) wird die Fließfähigkeit der Erdöldestillat-Brenn- bzw. Kraftstoffe erheblich beeinträchtigt. Die Paraffine verstopfen Filter und verursachen ungleichmäßige oder völlig unterbrochene Kraftstoffzufuhr zu den Verbrennungsaggregaten. Ähnliche Störungen treten bei Heizölen auf.

Es ist seit langem bekannt, daß durch geeignete Zusätze das Kristallwachstum der Paraffine in den Erdöldestillat Brenn- und Kraftstoffen modifiziert werden kann. Gut wirksame Additive verhindern einerseits, daß Mitteldestillate derartige Kartenhaus-Strukturen ausbilden und bei Temperaturen wenige Grad Celsius unterhalb der Temperatur, bei welcher die ersten Paraffinkristalle auskristallisieren, bereits fest werden und andererseits feine, gut kristallisierte, separate Paraffinkristalle bilden, welche Filter in Kraftfahrzeugen und Heizungsanlagen passieren oder zumindest einen für den flüssigen Teil der Mitteldestillate durchlässigen Filterkuchen bilden, so daß ein störungsfreier Betrieb sichergestellt ist.

Ein Nachteil dieses Standes der Technik beruht darauf, daß die ausgefallenen Paraffinkristalle aufgrund ihrer gegenüber dem flüssigen Teil höheren Dichte dazu neigen, sich beim Lagern mehr und mehr am Boden des Behälters abzusetzen. Dadurch bildet sich eine im oberen Behälterteil homogene paraffinarme Phase und am Boden eine zweiphasige paraffinreiche Schicht. Da sowohl in Fahrzeugtanks als auch in Lager- oder Liefertanks der Mineralölhändler der Abzug des Mitteldestillates meist wenig oberhalb des Behälterbodens erfolgt, besteht die Gefahr, daß die hohe Konzentration an festen Paraffinen zu Verstopfungen von Filtern und Dosiereinrichtungen führt. Diese Gefahr wird umso größer, je weiter die Lagertemperatur die Ausscheidungstemperatur der Paraffine (Trübungspunkt) unterschreitet, da die ausgeschiedene Paraffinmenge eine Funktion der Temperatur darstellt und mit sinkender Temperatur ansteigt.

Bei Kraftstoffen für OHo motoren treten diese Probleme nicht auf. Aus der DE-OS 26 24 630 ist es bekannt, Amide von Aminoalkylenpolycarbonsäuren als Zusatz für derartige Kraftstuffe zum Reinhalten von Einlaßventilen zu verwenden.

Bei den Paraffinkristallmodifikatoren für Mitteldestillate, den sog. Fließverbesserern, handelt es sich um Polymere, die durch Co-Kristallisation (Interaktion) das Kristallwachstum der n-Paraffine verändern. Dabei werden die Fließeigenschaften des Mitteldestillats bei niedrigeren Temperaturen positiv beeinflußt. Die Wirksamkeit der Fließverbesserer wird nach DIN 51428 indirekt durch Messung des "Cold Filters Plugging Points" ("CFPP") ausgedrückt.

Als Kältefließverbesserer werden an sich bekannte Ethylencopolymerisate, vor allem Copolymere von Ethylen und ungesättigten Estern, verwendet. DE 11 47 799 und 19 14 756 beschreiben beispielsweise Copolymerisate des Ethylens mit Vinylacetat, mit einem Gehalt von 25 bis 45 Gew.-% Vinylacetat oder Vinylpropionat und einem Molekulargewicht von 500 bis 5000.

Weiterhin ist aus GB 2 095 698 bekannt, Mitteldestillaten eine Kombination aus den genannten Copolymeren mit Amiden aus langkettigen Aminen und aromatischen oder cycloaliphatischen Carbonsäuren zuzusetzen.

Bezüglich der Dispergiereigenschaften der ausgeschiedenen Paraffine befriedigen diese Mischungen aber noch nicht.

Es bestand daher die Aufgabe, Zusätze zu Mitteldestillaten vorzuschlagen, die eine verbesserte Paraffindispergierwirkung bei guter Fließverbesserung besitzen.

Diese Aufgabe wurde mit neuen Umsetzungsprodukten, das sind Amide und/oder Amidammoniumsalze von Aminoalkylenpolycarbonsäuren mit sekundären Aminen der Formeln I und II

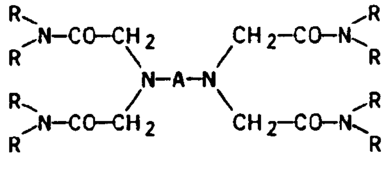

I

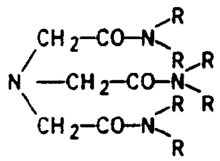

II

gelöst, in denen
A einen geradkettigen oder verzweigten Alkylenrest mit 2 bis 6 Kohlenstoffatomen oder den Rest der Formel

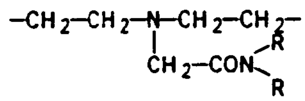

und
R im wesentlichen gradkettige aliphatische Reste, insbesondere Alkylreste mit 14 bis 24 C-Atomen bedeutet, wobei die Amidstrukturen auch zum Teil in Form der Ammoniumsalzstruktur der Formel

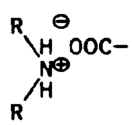

vorliegen können.

Die Amide bzw. Amid-Ammoniumsalze z.B. der Nitrilotriessigsäure, der Ethylendiamintetraessigsäure oder der Propylen-1,2-diamintetraessigsäure werden durch Umsetzung der Säuren mit 0,5 bis 1,5 Mol Amin, bevorzugt 0,8 bis 1,2 Mol Amin pro Carboxylgruppe erhalten.

Die Umsetzungstemperaturen betragen etwa 80 bis 200°C, wobei zur Herstellung der Amide eine kontinuierliche Entfernung des entstandenen Reaktionswassers erfolgt. Die Umsetzung muß jedoch nicht vollständig zum Amid geführt werden.

Als Amine der Formel

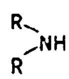

kommen insbesondere Dialkylamine in Betracht, in denen R einen geradkettigen Alkylrest mit 10 bis 30 Kohlenstoffatomen, vorzugsweise 14 bis 24 Kohlenstoffatomen, bedeutet. Im einzelnen seien Dioleylamin, Dipalmitinamin, Dikokosfettamin und Dibehenylamin und vorzugsweise Ditalgfettamin genannt.

Die neuen Amide bzw. Amidammoniumsalze der Aminoalkylenpolycarbonsäuren der Formel I und II werden den Erdölmitteldestillaten in Mengen von 50 bis 1000 ppm, bevorzugt 100 bis 500 ppm, zugesetzt. In der Regel enthalten die Mitteldestillate bereits Ethylen-Vinylester-Copolymerisate, insbesondere Ethylen-Vinylacetatcopolymerisate wie sie z.B. in DE 19 14 756 beschrieben sind.

Nach einer besonders bevorzugten Ausführungsform der Erfindung enthalten die Erdölmitteldestillatzusammensetzungen geringe Mengen der Komponenten der folgenden Additivkombination aus

a) 50-1000 ppm, bevorzugt 100-500 ppm der Aminoalkylenpolycarbonsäurederivate gemäß Formel I und/oder II

b) an sich bekannten Ethylencopolymerisat-Fließverbesserern, z.B Ethylen-Vinylestercopolymerisaten, in Mengen von 50-1000 ppm, bevorzugt 50 bis 500 ppm und

c) Leitfähigkeitsverbesserern in Form von Salzen, insbesondere von Carbonsäuren und sulfonsäuren bzw. deren Metall- und Ammoniumsalzen in Mengen von 0,25 bis 40 ppm, bevorzugt 1,5 bis 20 ppm

Die an sich bekannten Fließverbesserer (b) sind in der Patentliteratur eingehend beschrieben. Beispielsweise seien die Deutsche Patentschrift 19 14 756, EP 214786 (α-olefin/MSA-Ester) und EP 155807 (Alkylfumarate/VAC-Copolymere) genannt, auf die hiermit Bezug genommen wird. Es kommen jedoch auch gleichermaßen Terpolymerisate in Betracht, die neben Ethylen und Vinylestern oder Acrylestern noch weitere

Comonomere einpolymerisiert enthalten.

Bevorzugte Copolymerisate (b) sind solche, die im wesentlichen Ethylen und 25 bis45 Gew.-% Vinylacetat, Vinylpropionat oder Ethylhexylacrylat enthalten. Ferner sind Copolymerisate zu nennen, die beispielsweise Fumarsäureester enthalten. Das Molekulargewicht der Fließverbesserer beträgt in der Regel 500 bis 5000, vorzugsweise 1000 bis 3000.

Auch Mischungen verschiedener Fließverbesserer kommen in Betracht.

Als Leitfähigkeitsverbesserer (c) für Mitteldestillate kommen allgemein kohlenwasserstofflösliche Carbonsäuren und/oder Sulfonsäuren oder deren Salze in Betracht.

Die Grundleitfähigkeit von Mitteldestillaten beträgt ca. 5-10 ps/m, gemessen nach DIN 51 412. Schwankungen treten auf durch unterschiedliche Gehalte an Wasser, Salzen, Naphthensäuren, Phenolen und anderen schwefel- und stickstoffhaltigen Verbindungen.

Eine Anhebung der Leitfähigkeit um den Faktor 2 bis 3, bezogen auf die Grundleitfähigkeit, ist für das Dispergierverhalten der beschriebenen Paraffindispergatoren manchmal vorteilhaft.

Der Zusatz der Leitfähigkeitsverbesserer, wie sie z.B. in DE-OS 21 16 556 beschrieben sind, bewirken bereits in Mengen von 0,3 bis 1 ppm im Mitteldestillat eine Verbesserung des Ansprechverhaltens. Andere, weniger wirksame Leitfähigkeitsverbesserer erfordern naturgemäß eine höhere Konzentration. Der Zusatz deutlich größerer Mengen als der Beanspruchten ist zwar möglich, bringt aber keine wesentlichen technischen Vorteile.

Im einzelnen kommen ferner Metallsalze von kohlenwasserlöslichen Carbon- und Sulfonsäuren, wie sie sich unter der Bezeichnung ASA3/Shell im Handel befinden, sowie andere übliche Leitfähigkeitsverbesserer, wie das marktübliche Handelsprodukt Stadis 450 von DuPont, dessen Zusammensetzung nicht bekannt ist, in Betracht.

Die besondere Wirkung der genannten Kombination a+b+c ist überraschend und läßt sich aus den Eigenschaften der Komponenten nicht ableiten.

Die erfindungsgemäßen Verbindungen, gegebenenfalls zusammen mit den genannten weiteren Zusätzen können auch vorteilhaft in Form von flüssigen Konzentraten und in einfacherer Weise den Mitteldestillaten zudosiert werden.

Diese Konzentrate enthalten die Verbindungen der Formel I und/oder II in Mengen von 10 bis 60 Gew.%, gelöst in einem an sich bekannten Kohlenwasserstofflösungsmittel, z.B. Solvesso® der Firma Esso, unter Zusatz von mindestens 5 Gew.%, vorzugsweise 10 bis 20 Gew.%, bezogen auf die Gesamtmischung, eines Monoesters aus Phthalsäure und einem aliphatischen Alkohol von 6 bis 14 Kohlenstoffatomen, insbesondere 2-Ethylhexylphthalat.

Die Erfindung wird durch die folgenden Beispiele erläutert:

## Beispiele

### A) Herstellung der Nitriloessigsäureamide

1) 240 g (0.48 Mol) Ditalgfettamin und 35 g (0.12 Mol) Ethylendiaminotetraessigsäure wurden aufgeschmolzen und auf 190°C erhitzt, wobei das entstandene Reaktionswasser kontinuierlich abdestillierte. Die Umsetzung wurde nach ca. 25 Stunden bei einer Säurezahl < 10 und einer Aminzahl < 1.1 abgebrochen. Durch Anlegen von Wasserstrahlvakuum (2 Stunden, 120°C) wurde das Reaktionswasser vollständig entfernt. Man erhielt 265 g eines braunen, wachsartigen Feststoffes (= Paraffindispergator PD. (D) in den Tabellen).

2) 100 g (0.2 Mol) Ditalgfettamin und 14.6 g (0.05 Mol) Ethylendiaminotetraessigsäure wurden 8 Stunden auf 180°C erwärmt. Nach dieser Zeit waren ca. 50% des Amins zum Amid abreagiert (Säurezahl 45.8, Theorie 49.7). Man erhielt 97.6 g des Amid/Ammoniumsalzes als hellbraunen, wachsartigen Feststoff.

3) In eine Schmelze von 229.5 (0.45 Mol Ditalgfettamin wurden bei 80°C 28.65 g (0.15 Mol) Nitrilotriessigsäure (Trilon A) eingetragen. Anschließend wurde die Reaktionsmischung 10 Stunden auf 180 bis 190°C erhitzt. Zur vollständigen Entfernung des Reaktionswassers wurde das Produkt noch 2 Stunden bei 120°C am Wasserstrahlvakuum getrocknet. Man erhielt 249 g (Theorie 250 g) hellbraunen, wachsartigen Feststoff (= Paraffindispergator PD (H) in den Tabellen).

### B) Erdölmitteldestillatzusammensetzungen enthaltend

a) Nitriloessigsäureamide gemäß A),

b) als Fließverbesserer

FI (A) Ethylen/Vinylpropionat (mit ca. 40 Gew.% Vinylpropionat) mit einem mittleren Molekulargewicht von

ca. 2500 (Dampfdruckosmometrie)

FI (B) Ethylen/Vinylacetat (mit ca. 30 Gew.% Vinylacetat) mit einem mittleren Molekulargewicht von ca. 2500.

FI (C) Ethylen/Ethylhexylacrylat (mit 50 Gew.% Ethylhexylacrylat) mit einem mittleren Molekulargewicht von ca. 2500.

c) als Leitfähigkeitsverbesserer

LV. (E) (Kerostat 5009) gemäß Beispiel 1 der DE 21 16 556

LV. (F) (ASA 3/Shell) Kohlenwasserstofflösliches sulfocarbonsaures Salz

LV. (G) (Stadis 450/Du Pont) Leitfähigkeitsverbesserer unbekannter Zusammensetzung

Als Mitteldestillate wurden für die folgenden Versuche Heizöl EL und Dieselkraftstoff in handelsüblicher westdeutscher Raffineriequalität verwendet. Sie sind als Mitteldestillat A, B, C bezeichnet, wobei DK Dieselkraftstoff und HEL Heizöl EL bedeutet.

| | Mitteldestillat | | |
|---|---|---|---|
| | DK 1 (A) | DK 2 (B) | HEL (C) |
| Trübungspunkt (°C) | - 7°C | - 7°C | + 4°C |
| CFPP (°C) | - 10°C | - 13°C | - 1°C |
| Dichte b. 20°C (g/ml) | 0,817 | 0,827 | 0,826 |
| Siedeanfang (°C) | 156 | 165 | 171 |
| 20 % Siedepunkt (°C) | 204 | 210 | 218 |
| 90 % Siedepunkt (°C) | 309 | 318 | 344 |
| Siedeende (°C) | 350 | 358 | 369 |

Beschreibung der Testmethode

Die Mitteldestillate wurden mit unterschiedlichen Mengen an Fließverbesserern allein und/oder zusammen mit Paraffindispergatoren in Kombination mit Leitfähigkeitsverbesserer bei Temperaturen unterhalb des Trübungspunktes geprüft. Die Abkühlung erfolgte mit Hilfe eines Temperaturprogramms. Die Mitteldestillate A, B (Dieselkraftstoffe, Tabelle I, II) wurden dabei von Raumtemperatur auf -12°C mit einer Abkühlrate von 1°C/h abgekühlt und bei -12°C 24 h gelagert. Das Mitteldestillat C (Heizöl, Tabelle III) wurde ebenfalls von ca. 20°C mit 1°C/h auf -4°C abgekühlt und ebenfalls bei -4°C/24 h gelagert. Die Versuche wurden mit 100 ml und 1000 ml Mitteldestillatvolumina durchgeführt. In den Tabellen I-III sind aufgeführt: Volumen der sedimentierten Paraffinphase (%) (optisch bewertet), cloud point (CP) und cold filter plugging point (CFPP) in Bodennähe (untere 40 Vol.%), CP und CFPP des oberen Bereichs (obere 60 Vol.%) sowie der CP und CFPP des die Zusätze enthaltenden Mitteldestillates vor dem Lagertest.

Wie sich aus den folgenden Tabellen ergibt, wird durch Zusatz von leitfähigkeitsverbessernden Additiven die Sedimentation der Paraffine zusätzlich reduziert.

| | |
|---|---|
| Tabelle I: | Mitteldestillat A |
| Tabelle II: | Mitteldestillat B |
| Tabelle III: | Mitteldestillat C |
| Tabelle IV-VI: | Mitteldestillat A. |

In diesen Tabellen bedeuten

Art:

| | |
|---|---|
| T | = trüb |
| K | = klar |
| LD | = leicht dispergiert |
| D | = dispergiert |
| FI | = Mitteldestillatfließverbesserer (b) |
| PD | = Paraffindispergator (a) |
| LV | = Leitfähigkeitsverbesserer (c) |

Tabelle I Mitteldestillat A CP -7 °C/CFPP -10 °C

| FI (b) | | PD (a) | | LV | | Vor Lagerung | | Paraffin- | Paraffin/ | Untere Phase | | Obere Phase | |
|---|---|---|---|---|---|---|---|---|---|---|---|---|---|
| Typ | Konz. (ppm) | Typ | Konz. (ppm) | Typ | Konz. (ppm) | CP (°C) | CFPP (°C) | Sediment (Vol.%) | Oelphase | CP (°C) | CFPP (°C) | CP (°C) | CFPP (°C) |
| FI.(A) | 150 | - | - | - | - | -8 | -14 | 5 | T | -4 | -12 | -12 | -14 |
| FI.(A) | 300 | - | - | - | - | -8 | -18 | 12 | T | -4 | -12 | -11 | -17 |
| FI.(A) | 600 | - | - | - | - | -8 | -18 | 28 | K | -5 | -10 | -12 | -17 |
| FI.(A) | - | PD.(D) | 150 | - | - | -7 | -9 | 3 | T | -2 | -4 | -12 | -15 |
| FI.(A) | - | PD.(D) | 300 | - | - | -7 | -9 | 2 | T | -2 | -5 | -12 | -15 |
| FI.(A) | - | PD.(D) | 500 | - | - | -8 | -10 | 3 | LD | -4 | -7 | -11 | -15 |
| FI.(A) | - | - | - | LV.(E) | 1 | -8 | -9 | 37 | K | -1 | -5 | -12 | -15 |
| FI.(A) | - | - | - | LV.(E) | 5 | -7 | -9 | 39 | K | -2 | -5 | -12 | -16 |
| FI.(A) | 150 | PD.(D) | 150 | - | - | -8 | -15 | 2 | D | -4 | -12 | -10 | -20 |
| FI.(A) | 150 | PD.(D) | 150 | LV.(E) | 1 | -7 | -20 | 0 | D | -7 | -20 | -8 | -20 |
| FI.(A) | 150 | PD.(D) | 150 | LV.(E) | 5 | -8 | -19 | 0 | D | -7 | -20 | -9 | -20 |
| FI.(A) | 300 | PD.(D) | 300 | - | - | -8 | -29 | 23 | LD | -4 | -21 | -10 | -32 |
| FI.(A) | 300 | PD.(D) | 300 | LV.(E) | 1 | -7 | -29 | 0 | D | -7 | -29 | -8 | -30 |
| FI.(A) | 300 | PD.(D) | 300 | LV.(E) | 5 | -8 | -30 | 0 | D | -7 | -30 | -8 | -31 |
| FI.(A) | 300 | PD.(D) | 500 | - | - | -7 | -26 | 5 | LD | -5 | -22 | -10 | -30 |
| FI.(A) | 300 | PD.(D) | 500 | LV.(E) | 1 | -8 | -28 | 3 | D | -7 | -24 | -8 | -29 |
| FI.(A) | 300 | PD.(D) | 500 | LV.(E) | 5 | -8 | -29 | 0 | D | -8 | -27 | -9 | -29 |

Tabelle II Mitteldestillat B CP -7 °C/CFPP -13 °C

| FI(b) | | PD (a) | | LV | | Vor Lagerung | | Paraffin- | Paraffin/ | Untere Phase | | Obere Phase | |
|---|---|---|---|---|---|---|---|---|---|---|---|---|---|
| Typ | Konz. (ppm) | Typ | Konz. (ppm) | Typ | Konz. (ppm) | CP (°C) | CFPP (°C) | Sediment (Vol.%) | Oelphase | CP (°C) | CFPP (°C) | CP (°C) | CFPP (°C) |
| FI.(A) | 150 | - | - | - | - | -7 | -13 | 13 | T | -4 | -8 | -11 | -15 |
| FI.(A) | 300 | - | - | - | - | -7 | -13 | 18 | T | -4 | -9 | -11 | -15 |
| FI.(A) | 600 | - | - | - | - | -8 | -22 | 20 | T | -5 | -12 | -11 | -22 |
| - | - | PD.(D) | 150 | - | - | -8 | -13 | 83 D | T | -5 | -12 | -8 | -14 |
| - | - | PD.(D) | 300 | - | - | -8 | -15 | 87 D | T | -5 | -13 | -8 | -16 |
| - | - | PD.(D) | 500 | - | - | -8 | -15 | 87 D | T | -6 | -12 | -9 | -20 |
| - | - | - | - | LV.(E) | 1 | -7 | -12 | 18 | LD | -4 | -12 | -11 | -14 |
| - | - | - | - | LV.(E) | 5 | -8 | -13 | 20 | LD | -6 | -13 | -10 | -14 |
| FI.(A) | 150 | PD.(D) | 150 | - | - | -8 | -14 | 12 | LD | -4 | -9 | -8 | -19 |
| FI.(A) | 150 | PD.(D) | 150 | LV.(E) | 1 | -8 | -14 | 5 | D | -6 | -12 | -8 | -14 |
| FI.(A) | 150 | PD.(D) | 150 | LV.(E) | 5 | -8 | -15 | 2 | D | -8 | -12 | -9 | -16 |
| FI.(A) | 300 | PD.(D) | 300 | - | - | -7 | -21 | 10 | D | -5 | -12 | -9 | -28 |
| FI.(A) | 300 | PD.(D) | 300 | LV.(E) | 1 | -7 | -29 | 0 | D | -7 | -15 | -7 | -31 |
| FI.(A) | 300 | PD.(D) | 300 | LV.(E) | 5 | -8 | -28 | 0 | D | -8 | -26 | -9 | -29 |
| FI.(A) | 300 | PD.(D) | 500 | - | - | -8 | -28 | 0 | D | -7 | -19 | -8 | -30 |
| FI.(A) | 300 | PD.(D) | 500 | LV.(E) | 1 | -8 | -29 | 0 | D | -8 | -27 | -8 | -29 |
| FI.(A) | 300 | PD.(D) | 500 | LV.(E) | 5 | -8 | -28 | 0 | D | -8 | -28 | -9 | -30 |

Tabelle III Mitteldestillat C CP +4 °C/CFPP -1 °C

| FI (b) | | PD (a) | | LV | | Vor Lagerung | | Paraffin- | Paraffin/ | Untere Phase | | Obere Phase | |
|---|---|---|---|---|---|---|---|---|---|---|---|---|---|
| Typ | Konz. (ppm) | Typ | Konz. (ppm) | Typ | Konz. (ppm) | CP (°C) | CFPP (°C) | Sediment (Vol.%) | Oelphase | CP (°C) | CFPP (°C) | CP (°C) | CFPP (°C) |
| FI.(A) | 150 | - | - | - | - | +4 | -8 | 13 | T | +10 | +2 | -3 | -6 |
| FI.(A) | 300 | - | - | - | - | +3 | -10 | 20 | T | +10 | -4 | -2 | -10 |
| FI.(A) | 600 | - | - | - | - | +4 | -14 | 22 | T | +9 | -7 | -4 | -6 |
| - | - | PD.(D) | 150 | - | - | +3 | -1 | 90 | K | +7 | +3 | +3 | -2 |
| - | - | PD.(D) | 300 | - | - | +4 | ±0 | 90 | K | +8 | +2 | +3 | -4 |
| - | - | PD.(D) | 500 | - | - | +3 | -1 | 48 | K | +6 | +7 | -4 | -8 |
| - | - | - | - | LV.(E) | 1 | +4 | ±0 | 80 | K | +6 | +4 | +3 | -4 |
| - | - | - | - | LV.(E) | 5 | +4 | ±0 | 90 | K | +6 | +2 | +3 | -2 |
| FI.(A) | 150 | PD.(D) | 150 | - | - | +4 | -9 | 13 | T | +11 | ±0 | -2 | -6 |
| FI.(A) | 150 | PD.(D) | 150 | LV.(E) | 1 | +3 | -10 | 5 | LD | +10 | +1 | -1 | -7 |
| FI.(A) | 150 | PD.(D) | 150 | LV.(E) | 5 | +4 | -10 | 5 | LD | +9 | +4 | -3 | -8 |
| FI.(A) | 300 | PD.(D) | 300 | - | - | +4 | -10 | 18 | T | +10 | -6 | -2 | -9 |
| FI.(A) | 300 | PD.(D) | 300 | LV.(E) | 1 | +4 | -9 | 39 | D | +8 | -7 | +1 | -14 |
| FI.(A) | 300 | PD.(D) | 300 | LV.(E) | 5 | +4 | -8 | 2 | D | +5 | -11 | +3 | -11 |
| FI.(A) | 300 | PD.(D) | 500 | - | - | +4 | -11 | 20 | T | +8 | -7 | -1 | -8 |
| FI.(A) | 300 | PD.(D) | 500 | LV.(E) | 1 | +4 | -11 | 15 | D | +9 | -8 | +1 | -16 |
| FI.(A) | 300 | PD.(D) | 500 | LV.(E) | 5 | +4 | -10 | 0 | D | +5 | -11 | +2 | -15 |

Tabelle IV Mitteldestillat A CP -7 °C/CFPP -10 °C

| FI (b) | | PD (a) | | LV | | Vor Lagerung | | Paraffin- | Paraffin/ | Untere Phase | | Obere Phase | |
|---|---|---|---|---|---|---|---|---|---|---|---|---|---|
| Typ | Konz. (ppm) | Typ | Konz. (ppm) | Typ | Konz. (ppm) | CP (°C) | CFPP (°C) | Sediment (Vol.%) | Oelphase | CP (°C) | CFPP (°C) | CP (°C) | CFPP (°C) |
| FI.(B) | 300 | - | - | - | - | -8 | -28 | 10 | LD | -2 | -21 | -11 | -15 |
| FI.(B) | 300 | PD.(D) | 500 | - | - | -8 | -29 | 8 | LD | -4 | -27 | -10 | -33 |
| FI.(B) | 300 | - | - | LV.(E) | 2 | -7 | -27 | 10 | LD | -3 | -26 | -10 | -29 |
| FI.(B) | 300 | PD.(D) | 500 | LV.(E) | 2 | -8 | -32 | 0 | D | -7 | -30 | -7 | -28 |
| FI.(C) | 300 | - | - | - | - | -7 | -26 | 2 | LD | -2 | -24 | -11 | -31 |
| FI.(C) | 300 | PD.(D) | 500 | - | - | -7 | -26 | 0 | D | -7 | -24 | -7 | -31 |
| FI.(C) | 300 | - | - | LV.(E) | 2 | -7 | -25 | 8 | LD | -10 | -20 | -3 | -29 |
| FI.(C) | 300 | PD.(D) | 500 | LV.(E) | 2 | -8 | -25 | 0 | D | -7 | -23 | -7 | -26 |

Tabelle V Mitteldestillat A CP -7 °C/CFPP -10 °C

| FI (b) | | PD (a) | | LV | | Vor Lagerung | | Paraffin-Sediment | Paraffin/Oelphase | Untere Phase | | Obere Phase | |
|---|---|---|---|---|---|---|---|---|---|---|---|---|---|
| Typ | Konz. (ppm) | Typ | Konz. (ppm) | Typ | Konz. (ppm) | CP (°C) | CFPP (°C) | (Vol.%) | | CP (°C) | CFPP (°C) | CP (°C) | CFPP (°C) |
| FI.(A) | 300 | - | - | - | - | -8 | -18 | 12 | T | -4 | -12 | -11 | -17 |
| FI.(A) | 300 | PD.(H) | 500 | - | - | -8 | -25 | 0 | D | -7 | -23 | -8 | -31 |
| FI.(A) | 300 | PD.(H) | 500 | LV.(E) | 1 | -7 | -24 | 0 | D | -7 | -25 | -8 | -26 |
| FI.(A) | 300 | PD.(H) | 500 | LV.(E) | 5 | -8 | -26 | 0 | D | -7 | -26 | -8 | -28 |

Tabelle VI Mitteldestillat A CP -7 °C/CFPP -10 °C

| FI (b) Typ | FI (b) Konz. (ppm) | PD (a) Typ | PD (a) Konz. (ppm) | LV Typ | LV Konz. (ppm) | Vor Lagerung CP (°C) | Vor Lagerung CFPP (°C) | Paraffin-Sediment (Vol.%) | Paraffin/Oelphase | Untere Phase CP (°C) | Untere Phase CFPP (°C) | Obere Phase CP (°C) | Obere Phase CFPP (°C) |
|---|---|---|---|---|---|---|---|---|---|---|---|---|---|
| FI.(A) | 300 | - | - | LV.(F) | 1 | -8 | -18 | 12 | T | -3 | -20 | -12 | -16 |
| FI.(A) | 300 | - | - | LV.(F) | 5 | -7 | -19 | 12 | T | -3 | -20 | -11 | -18 |
| FI.(A) | 300 | PD.(D) | 500 | LV.(F) | 5 | -8 | -26 | 0 | D | -7 | -28 | -7 | -29 |
| FI.(A) | 300 | - | - | LV.(G) | 3 | -8 | -19 | 18 | T | -3 | -12 | -11 | -17 |
| FI.(A) | 300 | - | - | LV.(G) | 15 | -8 | -18 | 15 | T | -3 | -17 | -11 | -21 |
| FI.(A) | 300 | PD.(D) | 500 | LV.(G) | 15 | -7 | -30 | 0 | D | -7 | -27 | -7 | -33 |

## Patentansprüche

1. Amide und/oder Amidammoniumsalze der Formeln I und/oder II

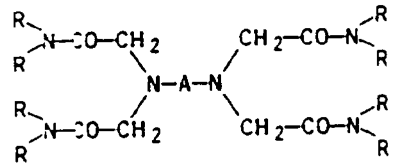

I

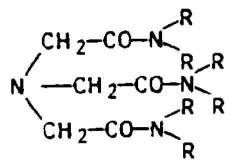

II,

wobei A einen geradkettigen oder verzweigten Alkylenrest mit 2 bis 6 Kohlenstoffatomen oder den Rest der Formel

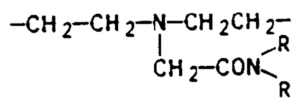

und
in denen R unabhängig voneinander im wesentlichen geradkettige aliphatische Reste mit 14 bis 24 C-Atomen, wobei anstelle der Amidgruppen zum Teil auch Alkylammoniumcarboxylat-Gruppen mit den genannten Resten R vorliegen können, bedeutet.

**2.** Verbindungen nach Anspruch 1 der Formel

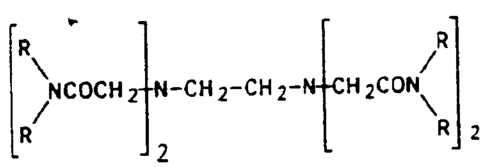

,

in der R einen geradkettigen Alkylrest mit 14 bis 24 Kohlenstoffatomen bedeutet und wobei anstelle der Amidgruppen auch zum Teil Dialkylammoniumcarboxylatgruppen der Amine

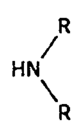

vorliegen können.

**3.** Flüssiges Konzentrat für die Zudosierung zu Mitteldestillaten, enthaltend 10 bis 60 Gew.%, bezogen auf das Konzentrat, von Verbindungen gemäß Anspruch 1 gelöst in einem Kohlenwasserstofflösungsmittel unter Zusatz von mindestens 5 Gew.%, bezogen auf das Konzentrat, an einem Monoester aus Phthalsäure und einem aliphatischen Alkohol mit 6 bis 14 C-Atomen.

**4.** Erdölmitteldestillatzusammensetzungen auf der Basis eines zwischen 160 und 420°C siedenden Kohlenwasserstoffgemisches, enthaltend eine als Paraffindispergator wirksame Menge der Verbindungen der Formeln I und/oder II

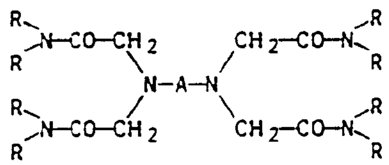

I

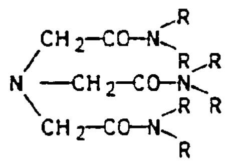

II,

wobei A einen geradkettigen oder verzweigten Alkylenrest mit 2 bis 6 Kohlenstoffatomen oder den Rest der Formel

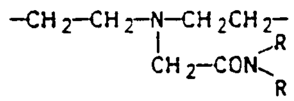

und
in denen R unabhängig voneinander im wesentlichen geradkettige aliphatische Reste mit 10 bis 30 C-Atomen, wobei anstelle der Amidgruppen zum Teil auch Alkylammoniumcarboxylat-Gruppen mit den genannten Resten R vorliegen können, bedeutet.

5. Mitteldestillatzusammensetzung gemäß Anspruch 4, enthaltend
   a) Amide und/oder Amidamoniumsalze gemäß Anspruch 4 sowie
   b) an sich bekannte als Mitteldestillat-Fließverbesserer geeignete Ethylencopolymere.

6. Mitteldestillatzusammensetzung gemäß Anspruch 4, enthaltend
   a) Amide und/oder Amidammoniumsalze gemäß Anspruch 4 in Mengen von 50 bis 1000 ppm,
   b) an sich bekannte als Mitteldestillat-Fließverbesserer geeignete Ethylencopolymere in Mengen von 50 bis 1000 pm und
   c) Kohlenwasserstoff-Leitfähigkeitsverbesserer, ausgewählt aus der Gruppe bestehend aus kohlenwasserstofflöslichen Carbonsäuren und Sulfonsäuren sowie deren Metall- und Ammoniumsalzen, in Mengen von 0,25 bis 40 ppm, jeweils bezogen auf die Zusammensetzung.

7. Mitteldestillatzusammensetzungen gemäß Anspruch 6, enthaltend die Komponente (a) in Mengen von 100 bis 500 ppm und die Komponente (b) in Mengen von 50 bis 500 ppm sowie die Komponente (c) in Mengen von 1,5 bis 20 ppm, bezogen auf die Zusammensetzung.

## Claims

1. An amide or amidoammonium salt of the formula I or II

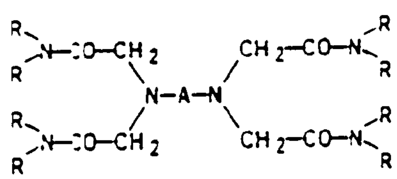

I

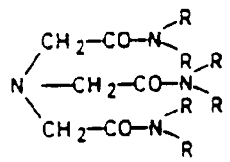

II

where A is straight-chain or branched alkylene of 2 to 6 carbon atoms or a radical of the formula

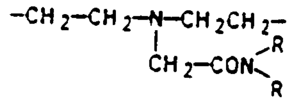

and

and the radicals R, independently of one another, are essentially straight-chain aliphatic radicals of 14 to 24 carbon atoms, and some of the amido groups may furthermore be replaced by alkylammonium carboxylate groups containing the stated radicals R.

**2.** A compound as claimed in claim 1, of the formula

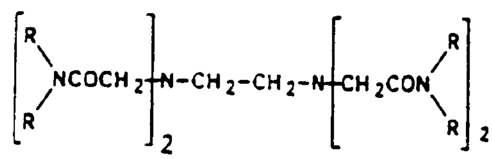

,

where R is straight-chain or branched alkyl of 14 to 24 carbon atoms and some of the amido groups may furthermore be replaced by dialkylammonium carboxylate groups of the amines

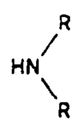

**3.** A liquid concentrate for metering into middle distillates, containing from 10 to 60% by weight, based on the concentrate, of compounds as claimed in claim 1, dissolved in a hydrocarbon solvent with the addition of at least 5% by weight, based on the concentrate, of a monoester of phthalic acid and an aliphatic alcohol with 6 to 14 carbon atoms.

**4.** A mineral oil middle distillate composition based on a hydrocarbon mixture boiling within a range from 160 to 420°C, containing the compounds of the formula I or II

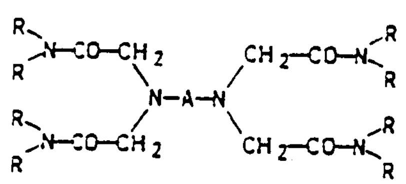

I

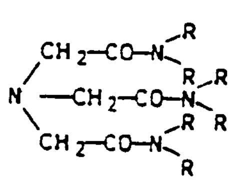

II

where A is straight-chain or branched alkylene of 2 to 6 carbon atoms or a radical of the formula

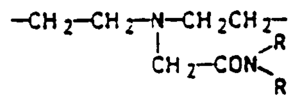

and

and the radicals R, independently of one another, are essentially straight-chain aliphatic radicals of 10 to 30 carbon atoms, and some of the amido groups may furthermore be replaced by alkylammonium carboxylate groups containing the stated radicals R, in an amount which is effective as a paraffin dispersant.

5. A middle distillate composition according to claim 4, containing
   a) amides or amidoammonium salts as claimed in claim 4 and
   b) conventional ethylene copolymers which are suitable as middle distillate flow improvers.

6. A middle distillate composition as claimed in claim 4, containing
   a) amides or amidoammonium salts as claimed in claim 4 in amounts of from 50 to 1,000 ppm, and
   b) conventional ethylene copolymers which are suitable as middle distillate flow improvers in amounts of from 50 to 1,000 ppm, and
   c) hydrocarbon conductivity improvers selected from the group consisting of hydrocarbon-soluble carboxylic acids and sulfonic acids and metal and ammonium salts thereof, in amounts of from 0.25 to 40 ppm, based in each case on the composition.

7. A middle distillate composition as claimed in claim 6, containing component (a) in amounts of from 100 to 500 ppm and component (b) in amounts of from 50 to 500 ppm and component (c) in amounts of from 1.5 to 20 ppm, based on the composition.

## Revendications

1. Amides et/ou sels d'amidammonium des formules I et/ou II

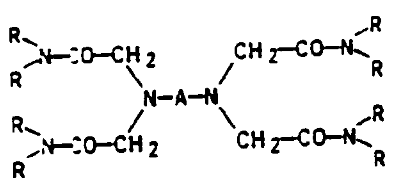

I

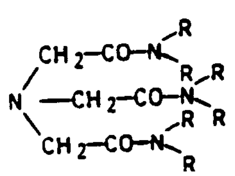

II,

où A représente un radical alkylène à chaîne droite ou à chaîne ramifiée, qui comporte de 2 à 6 atomes de carbone, ou le radical de la formule

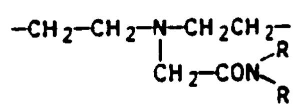

et

dans lesquelles les symboles R représentent, indépendamment les uns des autres, des radicaux aliphatiques essentiellement à chaîne droite, qui comportent de 14 à 24 atomes de carbone, où, au lieu des radicaux amide, peuvent aussi être partiellement présents des radicaux carboxylate d'alkylammonium avec les radicaux R précités.

2. Composés suivant la revendication 1 de la formule

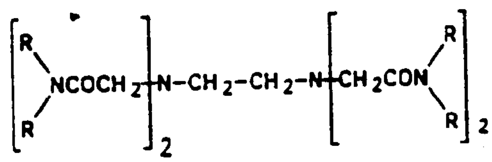

,

dans laquelle R représente un radical alkyle à chaîne droite, qui comporte de 14 à 24 atomes de carbone et où, au lieu des radicaux amide, peuvent également être présents des radicaux carboxylate de dialkyammonium des amines de la formule

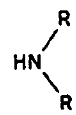

3. Concentré liquide destiné à être ajouté à des distillats moyens, contenant 10 à 60% en poids, par rapport au concentré, de composés suivant la revendication 1 dissous dans un solvant d'hydrocarbures, sous addition d'au moins 5% en poids, par rapport au concentré, d'un monoester de l'acide phtalique et d'un alcool aliphatique comportant de 6 à 14 atomes de carbone.

4. Compositions de distillat moyen du pétrole, à base d'un mélange d'hydrocarbures bouillant entre 160 et 420°C, qui contiennent, à titre d'agents dispersifs des paraffines, une proportion active des composés des formules I et/ou II

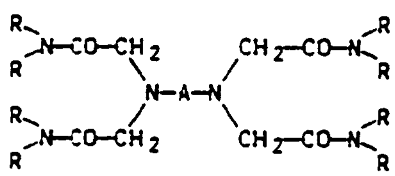

I

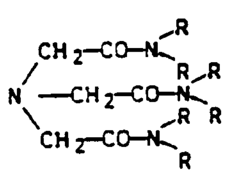

II,

où A représente un radical alkylène à chaîne droite ou à chaîne ramifiée, qui comporte de 2 à 6 atomes de carbone, ou le radical de la formule

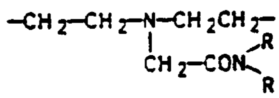

et
dans lesquelles les symboles R représentent, indépendamment les uns des autres, des radicaux aliphatiques essentiellement à chaîne droite, qui comportent de 14 à 24 atomes de carbone, où, au lieu des radicaux amide, peuvent aussi être partiellement présents des radicaux carboxylate d'alkylammonium avec les radicaux R précités.

**5.** Composition de distillant moyen suivant la revendication 4, qui contient
a) des amides et/ou des sels d'amidammonium selon la revendication 4, comme aussi
b) des copolymères d'éthylène connus, convenant comme agents d'amélioration de l'écoulement ou de la fluidité du distillat moyen.

**6.** Composition de distillat moyen suivant la revendication 4, qui contient
a) des amides et/ou des sels d'amidammonium selon la revendication 4 en proportions de 50 à 1000 ppm,
b) des copolymères d'éthylène connus, convenant comme agents d'amélioration de l'écoulement ou de la fluidité du distillat moyen, en proportions de 50 à 1000 ppm et
c) des agents d'amélioration de la conductibilité des hydrocarbures, choisis dans le groupe formé par des acides sulfoniques et des acides carboxyliques solubles dans les hydrocarbures, comme aussi leurs sels de métaux et d'ammonium, en proportions de 0,25 à 40 ppm, rapportées à chaque fois à la composition.

**7.** Compositions de distillat moyen selon la revendication 6, qui contient le composant (a) en proportions de 100 à 500 ppm et le composant (b) en proportions de 50 à 500 pm, comme aussi le composant (c) en proportions de 1,5 à 20 ppm, rapportées à la composition.